# EUROPEAN PATENT SPECIFICATION

(11) **EP 2 705 872 B1**
(45) Date of publication and mention of the grant of the patent: **10.08.2016**
(21) Application number: 12192484.9
(22) Date of filing: 13.11.2012
(51) Int. Cl.: A61M 25/02

(54) **Medical straps**
Medizinische Riemen
Sangles médicales

(30) Priority: 07.09.2012 TW 101217381
(43) Date of publication of application: 12.03.2014
(73) Proprietor: Taiwan Paiho Limited, Chang Hua Hsien (TW)
(72) Inventor: Cheng, Allen, _ Ho Mei Town, Chang Hua Hsien (TW)
(74) Representative: Lang, Christian

(56) References cited:
- DE-A1- 19 517 858
- US-A- 4 531 942
- US-A- 4 988 338
- US-A- 5 015 251
- US-A- 5 082 111
- US-A- 5 263 941
- US-A1- 2006 041 233
- US-B1- 7 793 892

## Description

### BACKGROUND OF THE INVENTION

### [Technical Field]

The present invention relates to a medical strap, and in particular to a medical strap for securing a medical tubing.

### [Description of the Prior Art]

Various tubings are often used in medical field. For medical and nursing techniques including injection, swathing, intubation, etc., tubings are necessarily used in injection and intubation for transmitting or storing liquid. In addition, tubings of aseptic materials are also frequently used for an medical operation, so that the surgery can be smoothly performed without causing any infection. In order to avoid displacements of these tubings due to the movement or the posture change of the patient, these tubings have to be carefully secured to minimize the possibility of falling off after a correct insertion of these tubings. Besides, some patients may pull or move these tubings when being injected and cause the transmission of liquid inside the tubings to fail. As a result, the tubings need to be secured over and over again in one single day to make sure the treatment can be continued. This will increase the loading of nursing staffs. Also, loose medical tubings may be medically troublesome for patients, and even cause other injuries to the patients. This may cause problems to medical safety.
US 7,793,892 B1 discloses a holder or organizer having first and second strips releasably fastened together by coupling material such as by hook and loop material. The strips each have two ends, namely a bonding end and a free end. The two bonding ends are coupled together. The first strip has an adhesive on its outside. The second strip has a selvedge area adjacent an edge, which selvedge area lacks releasable coupling material. The selvedge area will not fasten to the first strip. A user can grip the selvedge area of the second strip and peel the second strip off from the first strip. A tongue extends in the direction of the bonding ends of the strips and works in conjunction with a non-tongue area of the upper strip to hold an object in the holder.

### SUMMARY OF THE INVENTION

In order to solve the problems mentioned above, the main objective of the present invention is to provide a medical strap for securing a medical tubing.

According to the objective above, the present invention provides a medical strap as defined in the claims 1-4.

Another objective of the present invention is to provide a medical strap employing a combination of a securing portion and a movable portion for securing a medical tubing and reducing the displacement thereof.

Still another objective of the present invention is to provide a medical strap which is capable of simultaneously securing a plurality of medical tubings.

The medical trap proposed in the present invention is advantageous in reducing the displacement of the medical tubing during the securing process, and being capable of simultaneously securing a plurality of medical tubings. Adjustment may be made to the securing method according to the diameter of the medical tubing for optimizing the effect of securing the medical tubing.

### BRIEF DESCRIPTION OF THE DRAWINGS

FIG. 1 is a schematic view of a medical strap according to a first embodiment of the present invention.
FIG. 2 is a schematic view of a medical strap according to a second embodiment of the present invention.
FIG. 3 is a schematic assembled view of movable portions on the left half of the medical strap according to the first embodiment of the present invention.
FIG. 4 is a schematic assembled view of movable portions on the left half of the medical strap according to the second embodiment of the present invention.
FIG. 5 is a schematic view showing a process of securing a medical tubing with the medical strap according to the first embodiment of the present invention.
FIG. 6 is a schematic view showing the completed securing of the medical tubing using the medical strap according to the first embodiment of the present invention.
FIG. 7 is a schematic view showing a process of securing a medical tubing with the medical strap according to the second embodiment of the present invention.
FIG. 8 is a schematic view showing the completed securing of the medical tubing using the medical strap according to the second embodiment of the present invention.

### DETAILED DESCRIPTION OF THE PREFERRED EMBODIMENTS

Proposed is a medical strap having an upper strip and a lower strip. The upper and lower strips are bound together by joining a plurality of hooks and floss. The joining of the "hooks" and "floss" is achieved by the currently-available fastening techniques, so it will not be depicted in detail in the following description. In addition, the following drawings are not drawn to their actual dimensions, and serve as schematic views for illustrating the features of the present invention.

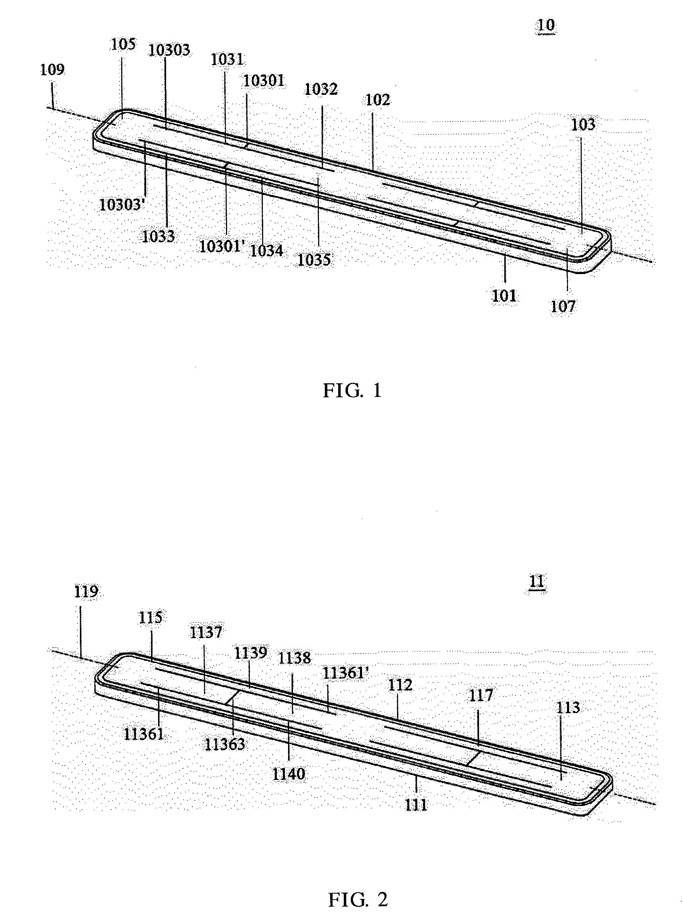

First, please refer to FIG. 1, which is a schematic view of a medical strap according to a first embodiment of the present invention. As shown in FIG. 1, the medical strap 10 of the present invention comprises a lower strip 101 and an upper strip 103. The lower strip 101 is opposingly attached to the upper strip 103. Two transverse or first parting lines 10303, 10303' and two longitudinal or second parting lines 10301, 10301' are provided on the left half 105 of the upper strip 103. The two transverse or first parting lines 10303, 10303' are of the same length and parallel to the longitudinal direction of the upper strip 103. The two transverse or first parting lines 10303, 10303' separate from each other by a distance, and are located respectively on both sides of a central line of symmetry 109 in the longitudinal direction of the upper strip 103. The two longitudinal or second parting lines 10301, 10301' are respectively perpendicular to the two transverse or first parting lines 10303, 10303', and respectively intersect two opposite edges 102 of the upper strip 103, thereby forming a securing portion 1035 and four movable portions 1031, 1032, 1033, 1034. The securing portion 1035 is located between the two transverse or first parting lines 10303 and 10303'. The movable portion 1031 is defined by the transverse or first parting line 10303, longitudinal or second parting line 10301 and an edge 102 of the upper strip 103. Two movable portions 1031 and 1032 are respectively located on both sides of the longitudinal or second parting line 10301, and two movable portions 1033 and 1034 are respectively located on both sides of the longitudinal or second parting line 10301'. A right half 107 symmetrical to the left half 105 also has a parting line structure which is similar to that of the left half 105, wherein the locations of the parting lines are similar to those mentioned above, and will not be described again. The lower strip 101 and upper strip 103 of this medical strap 10 are made of polymer materials. It is to be noted that the medical strap 10 is made without using bonding processes such as thermocompression bonding, ultrasonic bonding, or high frequency bonding.

Next, please refer to FIG. 2, which is a schematic view of a medical strap according to a second embodiment of the present invention. As shown in FIG. 2, a medical strap 11 of this embodiment comprises: a lower strip 111 and an upper strip 113. The lower strip 111 is opposingly attached to the upper strip 113. Two transverse or first parting lines 11361, 11361' and one longitudinal or second parting line 11363 are provided on the left half 115 of the upper strip 113. The two transverse or first parting ines 11361, 11361' are of the same length and parallel to the longitudinal direction of the upper strip 113. The two transverse or first parting lines 11361, 11361' separate from each other by a distance, and are located respectively on both sides of a central line of symmetry 119 in the longitudinal direction of the upper strip 113. The longitudinal or second parting line 11363 is perpendicular to and intersects the two transverse or first parting lines 11361, 11361', thereby forming two securing portions 1139 and 1140 as well as two movable portions 1137 and 1138. The two securing portions 1139 and 1140 are located respectively on both sides of the longitudinal or second parting line 11363. The two movable portions are located between the transverse or first parting lines 11361' and 11361 and an edge 112 of the upper strip 113, while the movable portions 1137 and 1138 are defined by the transverse or first parting lines 11361, 11361' and the longitudinal or second parting line 11363. A right half 117 symmetrical to the left half 115 also has a parting line structure which is similar to that of the left half 105, wherein the locations of the parting lines are similar to those mentioned above, and will not be described again. The lower strip 111 and upper strip 113 of this medical strap 11 are made of polymer materials. It is to be noted that the medical strap 11 is made without using bonding processes such as thermocompression bonding, ultrasonic bonding, or high frequency bonding.

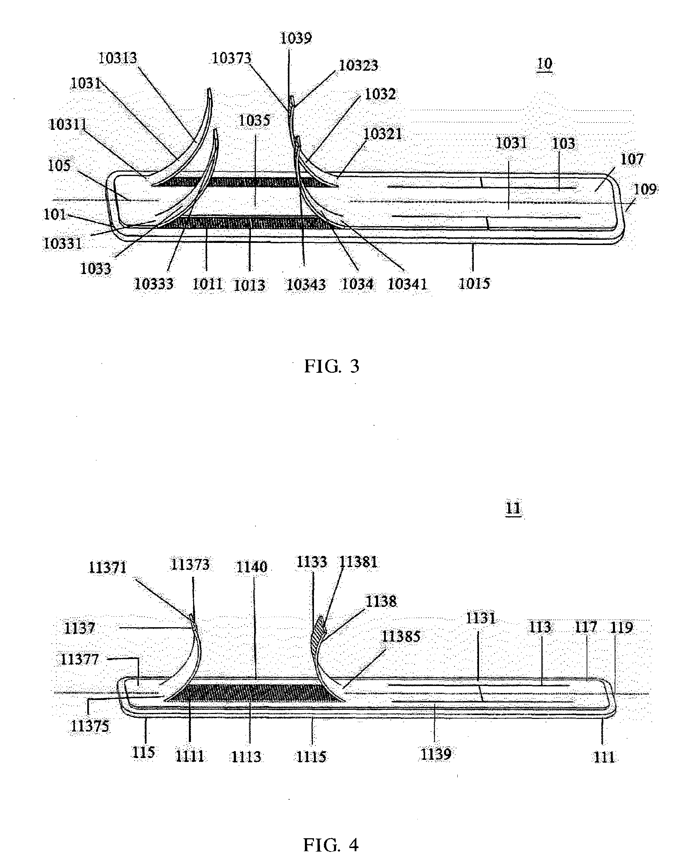

Next, please refer to FIG. 3, which is a schematic view of the movable portions on the left half of the medical strap of the first embodiment of the present invention. As shown in FIG. 3, the lower strip 101 has an upper surface 1013 and a lower surface 1015. The upper surface 1013 backs onto the lower surface 1015. A plurality of hooks 1011 are dispersed on the upper surface 1013. The lower surface 1015 is a smooth surface which may be provided with adhesive, by which the medical strap 10 is secured at a specific position. The upper strip 103 has an upper surface 1031 and a lower surface 1039. The upper surface 1031 backs onto the lower surface 1039. Floss 10373 is dispersed on the lower surface 1039. The floss 10373 on the lower surface 1039 of the upper strip 103 can be hooked by the hooks 1011 on the upper surface 1013 of the lower strip 101, so as to enable the attachment or join between the upper strip 103 and the lower strip 101. As mentioned above with respect to the first embodiment of the present invention, one securing portion 1035 and four movable portions 1031, 1032, 1033, 1034 are formed on the left half 105 of the upper strip 103, the two movable portions 1031 and 1032 are respectively located on both sides of the longitudinal parting line 10301, and the two movable portions 1033 and 1034 are respectively located on both sides of the longitudinal parting line : 10301'. One ends of the movable portions 1031, 1032, 1033 and 1034 are fixed ends 10311, 10321, 10331 and 10341, respectively, while the other ends opposite to the fixed ends 10311, 10321, 10331 and 10341 are free ends 10313, 10323, 10333 and 10343, respectively. The free ends 10313 and 10333 of the movable portions 1031 and 1033 are adjacent to the free ends 10323 and 10343 of the movable portions 1032 and 1034, respectively. The movable portions 1031, 1032, 1033 and 1034 have a structure similar to the upper strip 103, which is the same lower surface 1039. On the lower surface 1039 is the floss 10373 which can be hooked by the hooks 1011 on the upper surface 1013 of the lower strip 101 for attachment. In addition, the right half 107 on the side opposite to the left half 105 of the upper strip 103 also has a similar structure.

Next, please refer to FIG. 4, which is a schematic view of the movable portions on the left half of the medical strap according to the second embodiment of the present invention. As shown in FIG. 4, the lower strip 111 has an upper surface 1113 and a lower surface 1115. The upper surface 1113 backs onto the lower surface 1115. A plurality of hooks 1111 are dispersed on the upper surface 1113. The lower surface 1115 is a smooth surface which may be provided with adhesive, by which the medical strap 11 is secured at a specific position. The upper strip 113 has an upper surface 1131 and a lower surface 1133. The upper surface 1131 backs onto the lower surface 1133. Floss 11373 is dispersed on the lower surface 1133. The floss 11373 on the lower surface 1133 of the upper strip 113 can be hooked by the hooks 1111 on the upper surface 1113 of the lower strip 111, so as to enable the attachment between the upper strip 113 and the lower strip 111. As mentioned above with respect to the second embodiment of the present invention, two securing portions 1139, 1140 and two movable portions 1137, 1138 are formed on the left half 115 of the upper strip 113, the two movable portions 1137 and 1138 are respectively located on both sides of the longitudinal parting line 11363 and between the two securing portions 1139 and 1140. One ends of the movable portions 1137 and 1138 are fixed ends 11375 and 11385, respectively, while the other ends opposite to the fixed ends 11375 and 11385 are free ends 11371 and 11381, respectively. The free end 11371 is adjacent to the free end 11381. The movable portions 1137 and 1138 have a structure similar to the upper strip 113, which is the same lower surface 1133. On the lower surface 1133 is the floss 11373 which can be hooked by the hooks 1111 on the upper surface 1113 of the lower strip 111 for attachment. In addition, the right half 117 on the side opposite to the left half 115 of the upper strip 113 also has a similar structure.

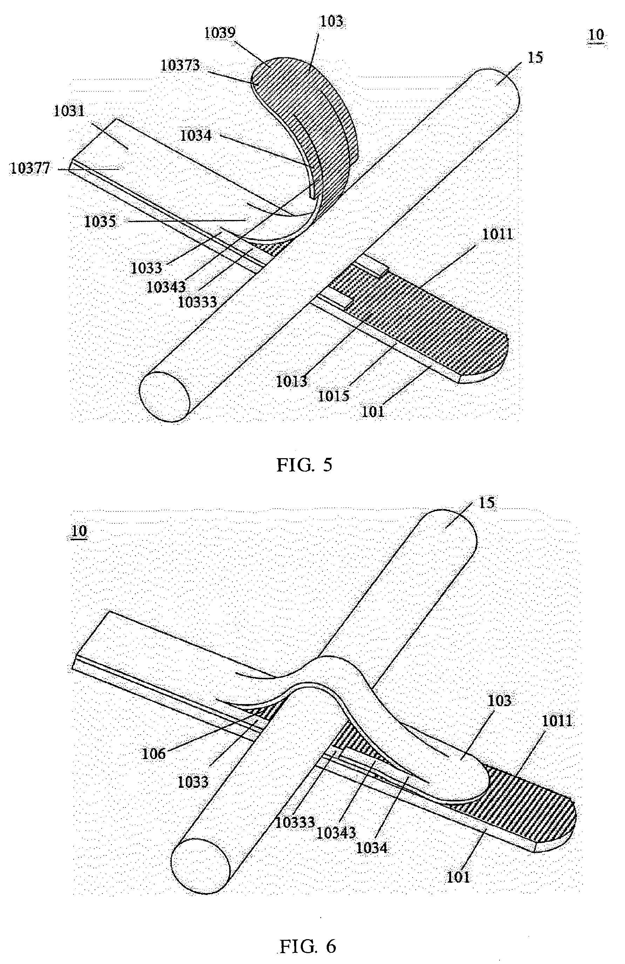

Next, please refer to FIG. 5 and FIG. 6, which are schematic views showing a process of securing a medical tubing with the medical strap according to the first embodiment of the present invention. As shown in FIG. 5, in the case of the movable portions 1033, 1034 and the securing portion 1035, for adjusting the tightness which is provided to the medical tube 15 by the securing portion 1035, adjustment can be made with the movable portions 1033, 1034 and the securing portion 1035 according to the diameter of a medical tubing 15, so that the displacement of the medical tubing 15 can be reduced. Specifically, at first, the upper strip 103 is not fully attached to the lower strip 101, and the movable portion 1034, which has a structure similar to the upper strip 103 and shares the same upper surface 1031, is not attached to the lower strip 101, either. Next, the medical tubing 15 is placed below the securing portion 1035 and above the upper surface 1031 of the movable portion 1033, on which a rough surface 10377 is provided. The rough surface 10377 is used for increasing the friction between the medical tubing 15 and the movable portion 1033, so that the movable portion 1033 can reduce the displacement of the medical tubing 15. Then, the movable portion 1034 is attached to the lower strip 101. Furthermore, since the lower surface 1039 of the securing portion 1035, the movable portion 1034 and the free end 10343 of the movable portion 1034 is the lower surface 1039 of the upper strip 103, the securing portion 1035, the lower surface 1039 of the movable portion 1034 and the free end 10343 of the movable portion 1034 is also provided with the floss 10373.

In the process of securing the medical tubing 15 with the medical strap 10, the floss 10373 provided on the lower surface 1039 of the securing portion 1035 will contact the medical tubing 15, and thereby clinging to the medical tubing 15. Next, the floss 10373 on the lower surface 1039 of the free end 10343 of the movable portion 1034 is hooked by the plurality of hooks 1011 provided on the upper surface 1013 of the lower strip 101 for attachment. Therefore, the free ends 10333, 10343 of the movable portions 1033, 1034 of the upper strip 103 will overlappingly engaged. In the formed structure, the movable portion 1034 is above the movable portion 1033, the securing portion 1035 is arched and thus forms a space 106 together with the lower strip 101. For securing the medical tubing 15, the user presses the securing portion 1035, so the floss 10373 provided on the lower surface 1039 of the securing portion 1035 is hooked by the hooks 1011 on the upper surface 1013 of the lower strip 101 for attachment. In such a way, the space 106 for the medical tubing 15 is gradually decreased, thereby securing the medical tubing 15 and reducing the displacement thereof. The secured medical tubing is shown in FIG. 6. Such method for securing the medical tubing 15 is also applicable for the combination of the other movable portions 1031, 1032 and the securing portion 1035. The aforementioned method for securing the medical tubing 15 is also applicable to the structure of the right half 107 that is a symmetric half of the left half 105. Adjustment may be made to the securing method according to the diameter of the medical tubing 15 for optimizing the effect of securing the medical tubing 15.

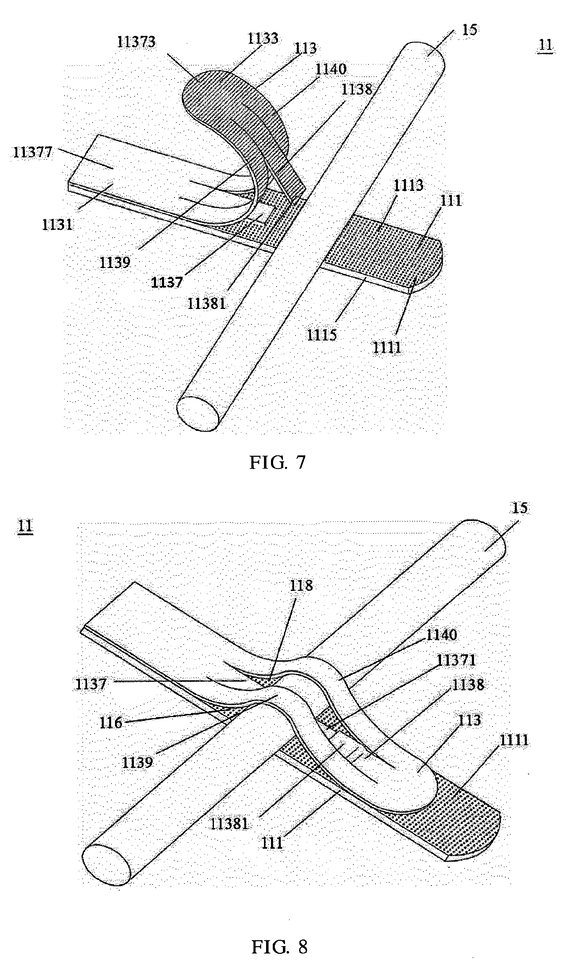

Next, please refer to FIGs. 7 and 8, which are schematic views showing a process of securing a medical tubing with the medical strap according to the second embodiment of the present invention. As shown in FIG. 7, in the case of the movable portions 1137, 1138 and the securing portions 1139 and 1140, for adjusting the tightness provided to the medical tube 15, adjustment can be made with the movable portions 1137, 1138 and the securing portions 1139, 1140 according to the diameter of a medical tubing 15, so that the displacement of the medical tubing 15 can be reduced. At first, the upper strip 113 is not fully attached to the lower strip 111, and the movable portion 1138 is not attached to the lower strip 111, either, wherein the movable portions 1137 and 1138 have a structure similar to the upper strip 113 and share the same upper surface 1131. Next, the medical tubing 15 is placed below the two securing portions 1139, 1140 and above the upper surface 1131 of the movable portion 1137, on which a rough surface 11377 is provided. The rough surface 11377 is used for increasing the friction between the medical tubing 15 and a contacting surface of the medical strap 11, so that the medical strap 11 can reduce the displacement of the medical tubing 15. Furthermore, since the lower surface 1133 of the securing portions 1139 and 1140, the movable portions 1137 and 1138, and the free ends 11371 and 11381 of the movable portions 1137 and 1138 is the lower surface 1133 of the upper strip 113, the securing portions 1139 and 1140, the movable portions 1137 and 1138, and the free ends 11371 and 11381 of the movable portions 1137 and 1138 is also provided with the floss 11373.

In the process of securing the medical tubing 15 with the medical strap 11, the floss 11373 provided on the lower surface 1133 of the securing portions 1139 and 1140 will contact the medical tubing 15, and the floss 11373 on the lower surface 1133 of the free end 11381 of the movable portion 1138 will also contact the medical tubing 15, therefore they are hooked by the plurality of hooks 1111 provided on the upper surface 1113 of the lower strip 111 for attachment. Thus, the free end 11381 of the movable portion 1138 of the upper strip 133 will partially overlappingly engaged with the free end 11371 of the movable portion 1137. In the formed structure, the free end 11381 is above the free end 11371, the securing portions 1139 and 1140 are arched and thus respectively form two spaces 116 and 118 together with the lower strip 111. For securing the medical tubing 15, the user presses the securing portions 1139 and 1140, so the floss 11373 provided on the lower surface 1133 is hooked by the hooks 1111 on the upper surface 1113 of the lower strip 111 for attachment. In such a way, the spaces 116 and 118 for the medical tubing 15 are gradually decreased, thereby securing the medical tubing 15 and reducing the displacement thereof. The secured medical tubing is shown in FIG. 8. The aforementioned method for securing the medical tubing 15 is also applicable to the structure of the right half 117 that is a symmetric half of the left half 115. Adjustment may be made to the securing method according to the diameter of the medical tubing 15 for optimizing the effect of securing the medical tubing 15.

## Claims

1. A medical strap (10, 11), comprising:
a first strip (101, 111) having opposingly arranged first surface (1013, 1113) and second surface (1015, 1115), a plurality of hooks (1011, 1111) being disposed on the first surface (1013, 1113), the second surface (1015, 1113) being a smooth surface; and
a second strip (103, 113) having opposingly arranged third surface (1039) and fourth surface (1031, 1131), a floss (10373, 11373) being disposed on the third surface (1039, 1133), the fourth surface (1031, 1131) being a rough surface,
wherein the floss (10373, 11373) on the third surface (1039, 1133) is hooked by the hooks (1011) on the first surface (1013, 1113) to join the first strip (101, 111) and the second strip (103, 113) together, and characterized
wherein a region on the second strip (103, 113) is provided with two first parting lines (10303, 10303', 11361, 11361') and at least one second parting line (10301, 10301', 11303), the first parting lines (10303, 10303', 11361, 11361') are of the same length, parallel to the longitudinal direction of the second strip (103, 113), spaced apart by a distance, and respectively located on both sides of a central line of symmetry (109, 119) of the second strip (103, 113), the at least one second parting line (10301, 10301', 11363) is perpendicular to one of the first parting lines (10303, 10303', 11361, 11361'), such that at least one securing portion (1035, 1139, 1140) and at least two movable portions (1031, 1032, 1033, 1034, 1137, 1138) are formed in the region on the second strip (103, 113), and the movable portions (1031, 1032, 1033, 1034, 1137,1138) are respectively located on both sides of the at least one second parting line (10301, 10301', 11363).

2. The medical strap (10) of claim 1, wherein the medical strap (10) has two second parting lines (10301, 10301'), wherein the two second parting lines (10301, 10301') respectively intersect two opposing edges of the second strip (103), and wherein one securing portion (1035) and four movable portions (1031, 1032, 1033, 1034) are formed in the region on the second strip (103), the securing portion (1035) is located between the first parting lines (10303, 10303'), and one of the movable portions (1031, 1032, 1033, 1034) is defined by one of the first parting lines (10303, 10303'), the second parting lines (10301, 10301') and an edge of the second strip (103).

3. The medical strap of claim 1, wherein the at least one second parting line (11363) intersects the first parting lines (11361, 11361'), and wherein two securing portions (1139, 1140) and two movable portions (1137, 1138) are formed in the region on the second strip (113), each of the securing portions (1139, 1140) is located between one of the first parting lines (11361, 11361') and an edge of the second strip (113), each of the movable portions (1137, 1138) is defined by the first parting lines (11361,11361') and the at least one second parting line (11363).

4. The medical strap (10) of claim 1, wherein the material of the first strip (101) and the second strip (103) is a polymeric material.

## Patentansprüche

1. Medizinisches Band (10, 11), umfassend:
einen ersten Streifen (101, 111) mit einer ersten Oberfläche (1013, 1113) und einer zweiten Oberfläche (1015, 1115), die gegenüberliegend angeordnet sind, wobei eine Vielzahl an Haken (1011, 1111) an der ersten Oberfläche (1013, 1113) vorgesehen ist und wobei die zweite Oberfläche (1015, 1113) eine glatte Oberfläche ist, und
einen zweiten Streifen (103, 113) mit einer dritten Oberfläche (1039) und einer vierten Oberfläche (1031, 1131), die gegenüberliegend angeordnet sind, wobei an der dritten Oberfläche (1039, 1133) eine Watte (10373, 11373) vorgesehen ist und wobei die vierte Oberfläche (1031, 1131) eine raue Oberfläche ist,
wobei die Watte (10373, 11373) an der dritten Oberfläche (1039, 1133) an den Haken (1011) an der ersten Oberfläche (1013, 1113) festgehakt ist, um den ersten Streifen (101, 111) und den zweiten Streifen (103, 113) miteinander zu verbinden, und
**gekennzeichnet dadurch, dass**
ein Bereich an dem zweiten Streifen (103, 113) mit zwei ersten Trennlinien (10303, 10303', 11361, 11361') und wenigstens einer zweiten Trennlinie (10301, 10301', 11363) ausgebildet ist, wobei die ersten Trennlinien (10303, 10303', 11361, 11361') die gleiche Länge haben, parallel zu der Längsrichtung des zweiten Streifens (103, 113) sind, durch einen Abstand voneinander getrennt sind und sich entsprechend auf den beiden Seiten von einer zentralen Symmetrielinie (109, 119) des zweiten Streifens (103, 113) befinden, wobei die wenigstens eine zweite Trennlinie (10301, 10301', 11363) senkrecht zu einer der ersten Trennlinien (10303, 10303', 11361, 11361') ist, sodass wenigstens ein Sicherungsabschnitt (1035, 1139, 1140) und wenigstens zwei bewegliche Abschnitte (1031, 1032, 1033, 1034, 1137, 1138) in dem Bereich an dem zweiten Streifen (103, 113) ausgebildet werden, wobei sich die beweglichen Abschnitte (1031, 1032, 1033, 1034, 1137, 1138) entsprechend auf den beiden Seiten von der wenigstens einen zweiten Trennlinie (10301, 10301', 11363) befinden.

2. Medizinisches Band (10) nach Anspruch 1, wobei das medizinische Band (10) zwei zweite Trennlinien (10301, 10301') hat, wobei die beiden zweiten Trennlinien (10301, 10301') entsprechend zwei gegenüberliegende Kanten des zweiten Streifens (103) schneiden, und wobei ein Sicherungsabschnitt (1035) und vier bewegliche Abschnitte (1031, 1032, 1033, 1034) in dem Bereich an dem zweiten Streifen (103) ausgebildet sind, wobei sich der Sicherungsabschnitt (1035) zwischen den ersten Trennlinien (10303, 10303') befindet, wobei einer der beweglichen Abschnitte (1031, 1032, 1033, 1034) jeweils durch eine der ersten Trennlinien (10303, 10303'), eine der zweiten Trennlinien (10301, 10301') und einen Rand des zweiten Streifens (103) definiert wird.

3. Medizinisches Band nach Anspruch 1, bei welchem die wenigstens eine zweite Trennlinie (11363) die ersten Trennlinien (11361, 11361') schneidet, wobei zwei Sicherungsabschnitte (1139, 1140) und zwei bewegliche Abschnitte (1137, 1138) in dem Bereich an dem zweiten Streifen (113) ausgebildet sind, wobei sich jeder der Sicherungsabschnitte (1139, 1140) zwischen einer der ersten Trennlinien (11361, 11361') und einem Rand des zweiten Streifens (113) befindet, und wobei jeder der beweglichen Abschnitte (1137, 1138) durch die ersten Trennlinien (11361, 11361') und die wenigstens eine zweite Trennlinie (11363) definiert ist.

4. Medizinisches Band (10) nach Anspruch 1, bei welchem das Material des ersten Streifens (101) und des zweiten Streifens (103) ein Polymermaterial ist.

## Revendications

1. Sangle médicale (10, 11) comprenant :
une première bande (101, 111) qui a une première surface (1013, 1113) et une seconde surface (1015, 1115) arrangées l'une en face de l'autre, une pluralité de crochets (1011, 1111) étant disposée sur la première surface (1013, 1113), la seconde surface (1015, 1115) étant une surface lisse et
une seconde bande (103, 113) qui une troisième surface (1039) et une quatrième surface (1031, 1131) arrangées l'une en face de l'autre, un tissu duveteux (10373, 11373) étant disposé sur la troisième surface (1039, 1133), la quatrième surface (1031, 1131) étant une surface rugueuse,
le tissu duveteux (10373, 11373) sur la troisième surface (1039) étant accroché par les crochets (1011) sur la première surface (1013, 1113) pour assembler ensemble la première bande (101, 111) et la seconde bande (103, 113) et **caractérisée en ce qu'**une région sur la seconde bande (103, 113) est pourvue de deux premières lignes de séparation (10303, 10303', 11361, 11361') et d'au moins une seconde ligne de séparation (10301, 10301', 11363), les premières lignes de séparation (10303, 10303', 11361, 11361') étant de la même longueur, parallèles à la direction longitudinale de la seconde bande (103, 113), espacées par une distance, et situées respectivement sur les deux côtés d'une ligne de symétrie centrale (109, 119) de la seconde bande (103, 113), la seconde ligne de séparation qui existe au moins (10301, 10301', 11363) étant perpendiculaire à l'une des premières lignes de séparation (10303, 10303', 11361, 11361') de telle manière qu'au moins une portion de fixation (1035, 1139, 1140) et au moins deux portions mobiles (1031, 1032, 1033, 1034, 1137, 1138) sont formées dans la région sur la seconde bande (103, 113) et les portions mobiles (1031, 1032, 1033, 1034, 1137, 1138) sont situées respectivement sur les deux côtés de la seconde ligne de séparation qui existe au moins (10301, 10301', 11363).

2. Sangle médicale (10) selon la revendication 1, la sangle médicale (10) ayant deux secondes lignes de séparation (10301, 10301'), les deux secondes lignes de séparation (10301, 10301') coupant respectivement deux bords opposés de la seconde bande (103) et une portion de fixation (1035) et quatre portions mobiles (1031, 1032, 1033, 1034) étant formées dans la région sur la seconde bande (103), la portion de fixation (1035) étant située entre les premières lignes de séparation (10303, 10303') et l'une des portions mobiles (1031, 1032, 1033, 1034) étant définie par l'une des premières lignes de séparation (10303, 10303'), les secondes lignes de séparation (10301, 10301') et un bord de la seconde bande (103).

3. Sangle médicale selon la revendication 1, la seconde ligne de séparation qui existe au moins (11363) coupant les premières lignes de séparation (11361, 11361') et deux portions de fixation (1139, 1140) et deux portions mobiles (1137, 1138) étant formées dans la région sur la seconde bande (113), chacune des portions de fixation (1139, 1140) étant située entre l'une des premières lignes de séparation (11361, 11361') et un bord de la seconde bande (113), chacune des portions mobiles (1137, 1138) étant définie par les premières lignes de séparation (11361, 11361') et la seconde ligne de séparation qui existe au moins (11363).

4. Sangle médicale (10) selon la revendication 1, le matériau de la première bande (101) et de la seconde bande (103) étant un matériau polymère.
